(19) Europäisches Patentamt / European Patent Office / Office européen des brevets

(11) **EP 3 869 948 B1**

(12) **EUROPEAN PATENT SPECIFICATION**

(45) Date of publication and mention of the grant of the patent:
**16.10.2024 Bulletin 2024/42**

(21) Application number: **19790004.6**

(22) Date of filing: **24.10.2019**

(51) International Patent Classification (IPC):
**A01K 41/06** $^{(2006.01)}$ **A01K 43/00** $^{(2006.01)}$
**G01N 33/08** $^{(2006.01)}$

(52) Cooperative Patent Classification (CPC):
**A01K 43/00; A01K 41/06; G01N 33/085; H05B 45/20;** Y02B 20/40

(86) International application number:
**PCT/EP2019/078982**

(87) International publication number:
**WO 2020/084036 (30.04.2020 Gazette 2020/18)**

(54) **CONTROLLER FOR CONTROLLING LIGHTING ELEMENTS**

STEUERGERÄT ZUR STEUERUNG VON BELEUCHTUNGSELEMENTEN

DISPOSITIF DE COMMANDE D'ÉLÉMENTS D'ÉCLAIRAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 EP 18202733**

(43) Date of publication of application:
**01.09.2021 Bulletin 2021/35**

(73) Proprietor: **Signify Holding B.V.**
**5656 AE Eindhoven (NL)**

(72) Inventors:
• **BROERSMA, Rémy C.**
**5656 AE Eindhoven (NL)**

• **SCHLANGEN, Lucas J. M.**
**5656 AE Eindhoven (NL)**
• **VAN DOOREN-SWEEGERS, Henrica I. A.J.**
**5656 AE Eindhoven (NL)**

(74) Representative: **Vanden Wyngaert, Hilbrand**
**Signify Netherlands B.V.**
**Intellectual Property**
**High Tech Campus 7**
**5656 AE Eindhoven (NL)**

(56) References cited:
**EP-A1- 2 982 224** **WO-A1-2018/160537**
**WO-A2-03/096028** **US-A1- 2011 228 515**
**US-A1- 2015 252 979**

**Description**

TECHNICAL FIELD

The present disclosure relates to a controller and lighting system for controlling the illumination emitted by one or more lighting elements within an environment.

BACKGROUND

Studies show that illuminating animal eggs (i.e. eggs containing unborn baby animals) during the development of the animals (e.g. baby birds) within their respective eggs (e.g. during incubation and hatching) may improve circadian rhythms before birth. A circadian rhythm is any biological process that follows a daily cycle of approximately twenty four hours. Circadian rhythms allow animals (including birds) to anticipate and prepare for regular environmental changes. For example, circadian rhythms are important in determining the sleeping and feeding patterns of animals. Circadian rhythms are often linked to light and darkness in an animal's environment. Illuminating animal eggs, in particular bird eggs, has also been shown to reduce stress levels, reduce incubation time and improve the development of the chicks.

A hatchery is a facility where eggs (e.g. bird eggs) are hatched, usually under artificial conditions. Bird hatcheries produce the majority of birds consumed by humans such as, amongst others, chickens, turkeys, ducks, and geese. Often no (natural or artificial) lighting is available in hatcheries and the like. This in turn means that the benefits of illuminating bird eggs discussed above are not realised. That is, having no light negatively affects the chicks' health and can increase mortality rates in newly hatched chicks.

In contrast, typically in the last few days of the eggs' hatching process, the eggs are subjected to constant illumination to enable the chicks to find food when they hatch from and leave their egg. Whilst some amount of illumination is beneficial for the chicks, being subjected to twenty four hours of illumination is not good for the circadian rhythm (which is linked to periods of light and darkness).

WO 2018/160537 A1 discloses methods of utilizing light during incubation of avian eggs to influence characteristics of avian pre- and post-hatch. Specific amounts of energy are provided to incubated eggs through a light source, having particular wavelengths, to accelerate embryo development and promote myogenesis post hatch. Green, red, or blue wavelengths of light, or combinations thereof, are provided to eggs in a temperature-controlled incubator to increase myoblast and satellite cell production in avian eggs. The light can be administered to avian eggs in a manner that entrains an embryo's circadian rhythm in the egg.

SUMMARY

The inventors of the present invention have realised that it would be beneficial to the health and development of unhatched animals to create periods of light and darkness as experienced by those animals (e.g. when inside an egg). The inventors have also realised that some animals require illumination at all times (e.g. during hatching so that when each animal hatches, those animals can find food, water, shelter, etc.). The inventors have also realised that illumination having different spectral compositions may be experienced by an animal as illumination having different intensities if placed after a filter such as, for example, by an unborn animal within the shell of an animal egg. The invention is defined by claims 1, 14 and 15. Other aspects of the invention are defined by the dependent claims.

According to a first aspect disclosed herein, there is provided a controller configured to control the illumination emitted by one or more light emitting elements within an environment for hosting one or more animals and a respective filter between each of the one or more animals and the one or more light emitting elements, wherein the controller is configured to: control the one or more light emitting elements to emit illumination having a first spectral composition for a first time period, wherein the first spectral composition is composed to produce, on a first side of the filter, a first level of illumination to create a period of relative lightness on the first side of the egg shell; control the one or more light emitting elements to emit illumination having a second, different spectral composition for a second, subsequent time period, wherein the second spectral composition is composed to produce, on a first side of the filter, a second, different level of illumination to create a period of relative darkness on the first side of the egg shell; and control the one or more light emitting elements to emit illumination throughout the first and second time periods to maintain, on a second side of the filter, a minimum level of illumination, wherein the one or more animals are one or more unhatched animals in respective unhatched animal eggs and the filter is the egg shell of the one or more unhatched animal eggs.

An advantage of this is that periods of relative lightness and relative darkness are created at the first side of the filter, such as inside the unhatched eggs, thus helping the entrainment (or creation) of a circadian rhythm before birth. For example, the first illumination level may be a lighter period and experienced as "daytime" whilst the second illumination level may be a darker period experienced as "night-time". Furthermore, the minimum illuminance level at the second side of the filter, such as within the environment outside the eggs, ensures that chicks hatching in either period (e.g. at night) are able to more easily find food, etc. Embodiments of the invention provide optimal lighting conditions for unborn as well as recently hatched chicks.

In embodiments, the first side of the filter may be the side between the animals and the filter, and the second side of the filter may be the side between the filter and the one or more light emitting elements.

In alternative embodiments, the second side of the filter may be the side between the animals and the filter, and the first side of the filter may be the side between the filter and the one or more light emitting elements.

In embodiments, the one or more animals may be birds (i.e. unhatched birds or chicks).

In embodiments, the one or more filters may be shell of unhatched animal eggs illuminated by the one or more light emitting elements, wherein the first spectral composition is composed to produce the first level of illumination inside the one or more unhatched animal eggs, wherein the second spectral composition is composed to produce the second, different level of illumination inside the one or more unhatched animal eggs, and wherein the illumination emitted throughout the first and second time periods maintains the minimum level of illumination outside the one or more unhatched animal eggs.

In embodiments, the one or more filters are the shell of unhatched animal eggs illuminated by the one or more light emitting elements, wherein the first spectral composition is composed to produce the first level of illumination outside the one or more unhatched animal eggs, wherein the second spectral composition is composed to produce the second, different level of illumination outside the one or more unhatched animal eggs, and wherein the illumination emitted throughout the first and second time periods maintains the minimum level of illumination inside the one or more unhatched animal eggs.

In embodiments, the one or more animals may be humans.

In embodiments, the one or more filters may be sunglasses illuminated by the one or more light emitting elements, wherein the illumination having the first spectral composition produces the first illumination level optically upstream the sunglasses (i.e. between the sunglasses and the light emitting elements), wherein the illumination having the second spectral composition produces the second, different illumination level optically upstream the sunglasses, and wherein the illumination emitted throughout the first and second time periods maintains the minimum illumination level optically downstream the sunglasses (i.e. between the sunglasses and the human's eyes).

In embodiments, the illumination emitted for the first time period may have a first egg transmittance factor and the illumination emitted for the second time period may have a second, different egg transmittance factor, wherein the egg transmittance factor is defined as a ratio of: (i) the amount of illumination that penetrates through the shell of the one or more unhatched eggs and (ii) the amount of illumination incident on the shell of the one or more unhatched eggs (which is linked to the amount of light emitted by the one or more light emitting elements).

In embodiments, the illumination emitted for the first time period may have a first egg transmittance per visible lumen and the illumination emitted for the second time period may have a second, different egg transmittance per visible lumen.

That is, more illumination penetrates the egg shell in one of the time periods compared to the other time period. This results in the chicks experiencing a period of relative lightness and a period of relative darkness.

In embodiments, the controller may be configured to control the one or more light emitting elements to emit illumination throughout the first and second time periods to maintain a constant illuminance level within the environment outside the one or more unhatched animal eggs.

An advantage of this is that the illumination level in the environment does not fluctuate and therefore that changes in illumination when switching from the first period to the second period, and vice versa, do not cause annoyance or unwanted distractions to the hatched chicks and/or any other user of the environment such as, for example, a farmer.

In embodiments, the controller may be configured to modify the luminous intensity of the illumination emitted by the one or more light emitting elements, wherein the illumination emitted for the first time period has a first luminous intensity and the illumination emitted for the second time period has a second, different luminous intensity.

The inventors have realised that varying the luminous intensity of the emitted illumination creates a second-order difference in illumination levels experienced within the unhatched animal eggs. That is, a period of relative lightness and relative darkness can be created during a first-order period of relative lightness and/or relative darkness (i.e. lighter lightness and darker lightness; and/or lighter darkness and darker darkness). Therefore, in embodiments, the controller may be configured to modify the luminous intensity of the illumination emitted by the one or more light emitting elements within the first time period and/or the second time period, wherein the illumination emitted in the first time period may have a first luminous intensity during a first subperiod of said first time period and a second, different from the first, luminous intensity during a second subperiod of said first time period and/or the illumination emitted in the second time period may have a third luminous intensity during a third subperiod of said second time period and a forth, different from the third, luminous intensity during a forth subperiod of said second time period.

In embodiments, the controller may be configured to: control the one or more light emitting elements to emit the illumination in the first time period with a first colour temperature; and control the one or more light emitting elements to emit the illumination in second time period with a second, different colour temperature.

In embodiments, the first colour temperature may be greater than the second colour temperature such that the first illumination level inside the one or more unhatched animal eggs is less than the second illumination level inside the one or more unhatched animal eggs.

In the context of this description, colour temperature of illumination or colour temperature of a light emitting element is the temperature of an ideal black-body radiator that radiates light of a colour comparable to that of the light emitting element. Colour temperature is a characteristic of visible light and is most often associated with "white light". White light is defined herein as light having chromaticity coordinates within seven or eight MacAdam ellipses from the black body locus (BBL). Colour coordinates that lie on or near the BBL yield pleasing white light to a human observer. However, many artificial light emitting elements, such as fluorescent lamps or LEDs (light emitting diodes) emit light primarily by processes other than thermal radiation which means that the emitted radiation does not follow the black body locus spectrum. These sources are assigned what is known as a correlated colour temperature CCT. CCT is the colour temperature of a black body radiator which, to human colour perception, most closely matches the light from the artificial light emitting element. General illumination generally has a (correlated) colour temperature between 2000 K and 10000 K, with the majority of lighting emitting elements for general illumination being between 2700 K and 6500 K.

The inventors have realised that in contrast to what is normally experienced in the environment outside of the unhatched animal eggs, warmer illumination (e.g. warm white light with a low (correlated) colour temperature) creates relative lightness inside the unhatched animal eggs and colder illumination (e.g. cold white light with a high (correlated) colour temperature) creates relative darkness inside the unhatched animal eggs.

In embodiments, the controller may be configured to: control the one or more light emitting elements to emit the illumination in the first time period having a same (correlated) colour temperature for the first time period; and control the one or more light emitting elements to emit the illumination in the second time period having the same (correlated) colour temperature for the second time period.

In embodiments, the controller may be configured to control the one or more light emitting elements to emit a light-dark cycle of illumination, wherein the light-dark cycle of illumination comprises a plurality of contiguous time periods of illumination comprising the first and second time periods of illumination, wherein the illumination emitted for each respective time period has a different spectral composition and/or a different luminous intensity compared to the illumination emitted for the other respective time periods in the light-dark cycle.

For example, the light-dark cycle may be used to create or entrain a circadian rhythm during hatching. The light-dark cycle may comprise relatively lighter periods having different illumination levels and relatively darker periods having different illumination levels.

In embodiments, the controller may be configured to control the one or more light emitting elements to emit the light-dark cycle of illumination over a time period of one day.

An advantage of this is that such light-dark cycle may mirror the natural light and dark periods created by sunlight and moonlight over a twenty-four hour period.

In embodiments, the time periods may be programmable by an end-user.

In embodiments, the light-dark cycle of illumination may comprise at least one further time period of illumination having a third, different spectral composition and/or a third, different luminous intensity compared to that of the first and second time periods.

For example, the further time period having the third spectral composition and/or third, different luminous intensity may result in an illumination level within the unhatched animal eggs in between the illumination level caused by the first time period of illumination and the second time period of illumination.

In embodiments, the illumination emitted by the one or more light emitting elements may be visible illumination.

According to second aspect disclosed herein, there is provided a system comprising: a controller according to any of the claimed embodiments disclosed herein and one or more light emitting elements configured to emit illumination into the environment.

In an embodiment, the system may comprise a filter adapted to be disposed between the lighting elements and each animal.

In embodiments, the system may also comprise an animal housing unit for hosting an animal, and wherein the controller is configured to control the one or more light emitting elements to emit illumination in the animal housing unit, such that the animal, when hosted in the housing unit, is illuminated by said illumination.

According to a third aspect disclosed herein, there is provided a computer program product comprising code embodied on computer-readable storage and configured so as when run on one or more processing units of a control system to control one or more light emitting elements to perform operations in accordance with any of the claimed embodiments disclosed herein.

BRIEF DESCRIPTION OF THE DRAWINGS

To assist understanding of the present disclosure and to show how embodiments may be put into effect, reference is made by way of example to the accompanying drawings in which:

Figure 1 shows an example of the egg transmittance through a typical chick egg shell at different wavelengths,
Figure 2 shows an example comparison of the relative photopic eye sensitivity of humans and chickens,
Figure 3 shows a first graph depicting how egg transmittance factor changes with colour temperature,
Figure 4 shows a second graph depicting how egg transmittance factor changes with colour temperature,
Figure 5 shows an example graph depicting the change in intensity for high and low egg transmittance factor spectra at the same colour temperature.
Figure 6 shows schematically an environment comprising a lighting system for illuminating the environment,
Figure 7 shows schematically a block diagram of a control system for controlling a lighting system, and
Figure 8 shows schematically an example light-dark illumination cycle,

DETAILED DESCRIPTION

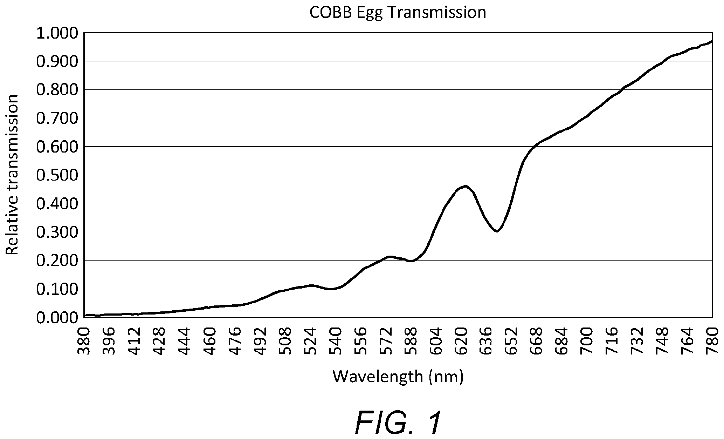

The transmission of egg shells is spectrally dependent. The transmission of the eggshell of a typical Cobb chick is shown in Figure 1. As shown in Figure 1, the relative transmission of illumination through an egg shell generally increases as the wavelength of illumination increases, with some variance between approximately 540 to 650 nm. Therefore as the wavelength of illumination increases, more light passes through the egg shell and therefore the unhatched chicks are subjected to greater illumination levels.

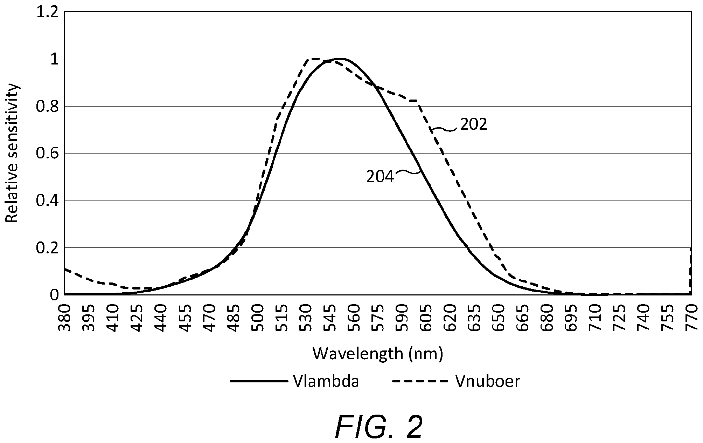

Lumens (Lm) is a measurement of the total amount of light as perceived by the human visual system that is emitted from a light source (i.e, the luminous flux), whereas illuminance (Lux) is a measurement of the amount of light as perceived by the human visual system that is incident on a given area - one lux is equal to one lumen per square meter. According to Nuboer et al. (Nuboer, J. F. W., et al. (1992). "Artificial lighting in poultry houses: Are photometric units appropriate for describing illumination intensities?" British Poultry Science 33(1): 135-140), Figure 2 illustrates a typical chicken eye sensitivity curve. When multiplying the visual sensitivity function for chicken with the spectral radiance of a light source one obtains the chicken equivalent of the human (photopic) luminous flux, for convenience expressed here "chicken-Lm".

Equally, for any animal the visual sensitivity function for that animal multiplied with the spectral (ir) radiance defines the "animal-lm" or "animal-lx" to specify the "animal (il) luminance".

The "chicken Lm"/"chicken-Lux" levels of a light source after filtering (i.e. by an egg shell) as observed by a chick and normalized to the Lm/Lux level of a light source before filtering as observed by a human. Here, the light before filtering can be the lm/lux (for a human observer), but it can also be the "chicken lux" or "chicken lumen" (for a chicken observing the light).

In Fig. 2 the eye sensitivity for chicks is shown by curve 202 and the eye sensitivity for humans is shown by curve 204. Egg transmission factor (ETF) defines the ratio of the Lm/Lux in the egg compared to the amount of Lm/Lux of the light source, and is defined by:

$$ETF = \frac{683 \int_{380}^{780} \phi\lambda \times Tregg \times vn \, d\lambda}{683 \int_{380}^{780} \phi\lambda \times v \, d\lambda}$$

$\varphi\lambda$ = Spectral distribution (radiance or irradiance) of light source (W/nm or W /nm m2)

Tregg = Spectral transmission of (egg) filter
$v\,n$ = Spectral Eye sensitivity of chicken
$v$ = Spectral Eye sensitivity of human

The denominator in the previous equation gives the photopic lumen (or lx) for a human, and the numerator gives the "chicken lumen" (or "chicken lx") within the egg. ETF varies depending on the spectral composition of the illumination emitted by a light source.

The observers on the two sides can, but need not, be of the same species: on one side of the filter the observer can be a human and on the other side an aminal (e.g. a chicken). In that case, the illumination level experienced by the human is expressed in lx for humans, and the illumination level experienced by the animal in "animal-lx" (like, for instance, "chicken-lx" as follows from Nuboer et al.).

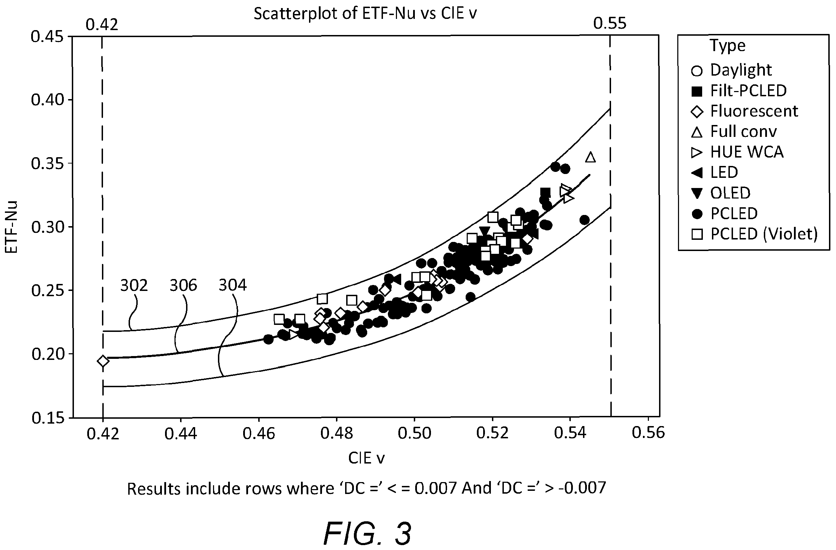

Figure 3 illustrates an ETF graph for a variety of light sources for a bandwidth of ETF values defined by: v' between 0.42 and 0.55

$$\text{ETF average} = -2.392 + 19.33 * v' - 48.43 * v'^2 + 40.66 * v'^3$$

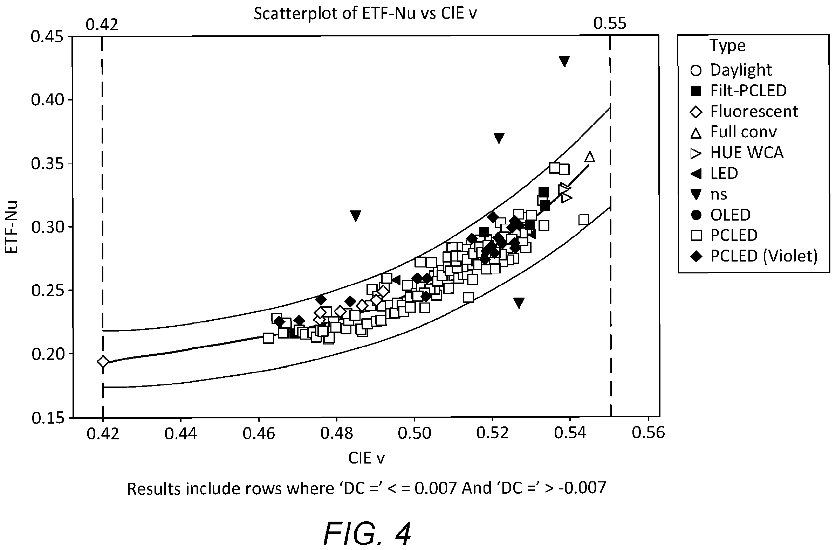

The upper (curve 302) and lower (curve 304) limits are defined as +11% and - 11% respectively of the ETF average (curve 306). The colour coordinates noted in relation to Figures 3 and 4 are defined according to the 1976 CIE u'v' color definition.

Figure 3 shows that spectra having a higher v' (corresponding to a lower colour corrected temperature) have a higher lux level in the egg than spectra having a lower v' (higher colour corrected temperature) at the same lux level outside of the egg. This means that if the colour point outside of the eggs is changed, while keeping the illumination level (Lux) constant, a light-dark profile is created within the egg. The dynamic range between v' =0.42 and v' = 0.55 results in a factor of two in perceived brightness. In the example of Figure 3, the light sources were restricted to white light sources with a maximum deviation on average of du'v' of 0.007 from the black body locus (BBL). Furthermore, the amplitude can be amplified in a secondary order to also add an intensity variation. The light-dark cycle observed within the egg remains different compared to outside the egg. Therefore, this would mean that for the lighter period a warmer colour is preferred, while for the darker period a cooler colour is preferred. This is in contrast to what is currently seen as circadian, where the lighter period has a cooler colour and the darker period has a warmer colour.

The dynamic range can be enhanced or different white colours may be used if the spectrum of the light source can be adapted to ETF values beyond current boundaries as defined and depicted in figure 3. Figure 4 illustrates the ETF values of new spectra (ns), shown by inverted triangles, that have been realized that have significantly higher or lower ETFs than current white LEDs. A greater dynamic range is used to increase and decrease the illumination level within the egg shell (larger vertical distance in Fig. 4 between first spectral composition and second spectral composition), and/or to create a light-dark cycle with cooler colours in the light period and warmer colours in the dark period (larger horizontal distance in Fig. 4 between first spectral composition and second spectral composition).

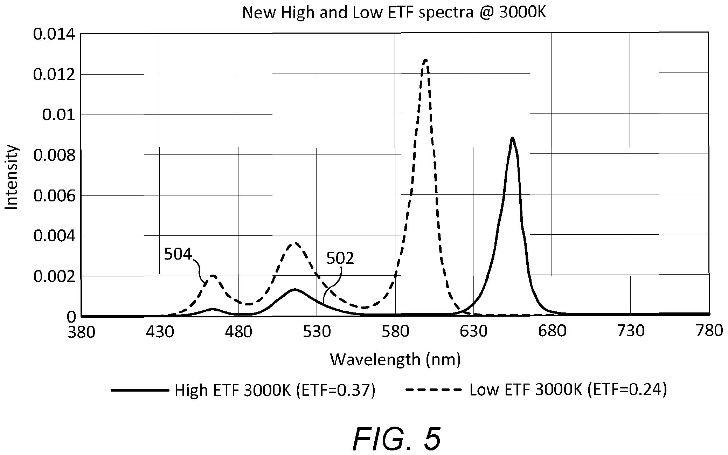

Figure 5 illustrates an example of the newly defined spectra (ns) for high (curve 502) and low (curve 504) ETF solutions at a v' of ~ 0.523 and a colour temperature of 3000K. As shown from Figure 5, variations in intensity within the eggshell can be produced for a constant colour temperature.

Embodiments of the invention will now be described by way of example.

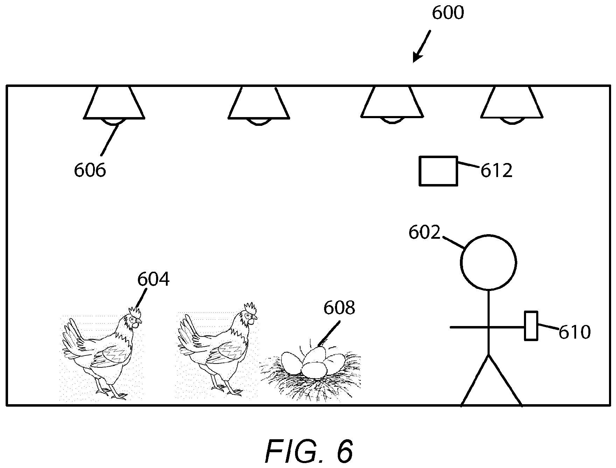

Figure 6 illustrates an example environment 600 in which embodiments disclosed herein may be employed. The environment 600 is a space that may be occupied by one or more animals (e.g. human users 602 and/or one or more birds 604). In general, the animals may be any member of the Animalia kingdom. The animals are living animals. The environment contains one or more unhatched eggs 608. For example, the eggs may be eggs of birds such as chickens, turkeys, pheasants, geese, fowl, Guinea fowl, quail, etc. In general, the eggs may be eggs of any egg-laying animal. For example, the eggs may be eggs of birds, lizards, snakes, reptiles, amphibians, etc. The environment 600 may take the form of an indoor space such as one or more rooms of a home, office or other building such as a barn, hatchery or farmhouse; an outdoor space such as a garden or park; a partially covered space such as a gazebo; or a combination of such spaces such as a farm comprising both indoor and outdoor spaces. The environment 600 may also take the form of a vivarium or terrarium.

The environment 600 is equipped with a plurality of (optical) light emitting elements (or lighting elements, or light sources, or luminaires) 606 installed or otherwise disposed at different locations throughout the environment 600. A light emitting element 606 may refer to any kind of light emitting device for illuminating an environment or part of the environment occupied by a user 602 or bird 604, whether providing ambient lighting or task lighting. Each of the light emitting elements 606 may take any of a variety of possible forms, such as a ceiling or wall mounted luminaire, a free-standing floor or table light source 606, an light source 606 mounted on a pole, gantry or rigging (and the different light sources 606 in the environment 600 need not take the same form as one another). Whatever form it takes, each light emitting element 606 comprises at least one lamp (light element) and optionally any associated housing, socket and/or support. Examples of suitable lamps include LED-based lamps, or traditional filament bulbs or gas discharge lamps. In examples, the one or more light emitting elements 606 may be a single type of light emitting diode (LED) or multiple types of LEDs. In examples, the LEDs may be phosphor converted LEDs or direct LEDs and may emit light in the range of ultraviolet to infrared. It will be appreciated that the light emitting elements 606 may be light emitting elements other than LEDs.

The environment 600 comprises a plurality of filters between each of the animals and the light emitting elements (i.e. a respective filter for each animal). In the case of the animal being a human, the filter may be, in general, an object that allows some light to pass through it such as, for example, a pair of glasses or sunglasses. In the case of the animal being non-human, the filter may be the egg shell in which the unhatched animal is contained.

In some scenarios the environment 600 may be divided into a plurality of different zones or localities (not shown), such as different rooms, each illuminated by a different respective subset of one or more of the light emitting elements 606. For example, the different zones may relate to different sections of a farmhouse or bird coup.

The environment 600 may also be equipped with one or more lighting control devices 610. Each of the lighting control devices 610 may take the form of a stand-alone lighting control device 610 such as a smart light switch, a dimming switch, etc. or alternatively a lighting control device 610 integrated in another user device such as a mobile user terminal such as a smartphone or tablet, or even a wearable device that can be worn about the user's person. For example, the user terminal may be installed with a suitable lighting control app. The lighting control device 610 can be mains powered, battery powered, or use energy-harvesting techniques to supply its energy. The lighting control device 610 is configured to be able to control the light emitted by one or more light emitting elements 606 in the environment 600. This may include switching the light emitting elements 606 on/off, controlling the colour of the light, controlling the dimming level, controlling a time-varying effect of the light, controlling a spatial-varying effect of the light or adjusting any other aspects of the light that may be applicable to the light emitting elements 606 within the environment 600. The environment 600 may also be equipped with a central lighting system bridge or hub 612.

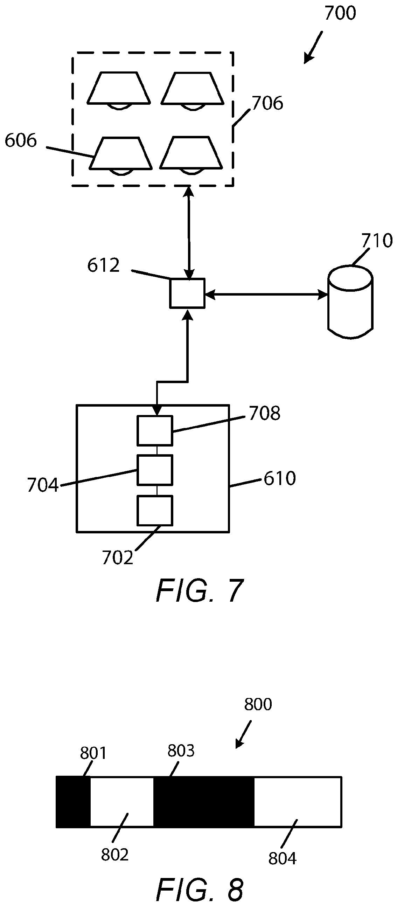

Figure 7 illustrates a control system 700 enabling the user 602 to control the light from one or more of the light emitting elements 606 based on one or more inputs from the user 602 to a lighting control device 610 and/or control the light state of the light emitting elements 606. This may be to control the light in the same part of the environment 600 as that in which the user 602 inputs a command to the lighting control device 610 or a different part of the environment 600.

A server/database 710 is shown in Figure 7. The database is configured to store one or more settings associated with the one or more light emitting elements 606. For example, the database 710 may store program settings, or settings defining time periods of illumination detailed herein. The database 710 may alternatively be stored on the lighting control device 610, a central lighting system bridge 612, or across one or more light emitting elements 606.

The lighting control device 610 comprises a user interface 702 arranged to receive an input from the user 602 and operatively coupled to a controller 704. The user interface 702 may comprise a display in the form of a screen and means for receiving inputs from the user. For example, the user interface 702 may comprise a touch screen, or a point-and-click user interface comprising a mouse, track pad, or tracker ball or the like. Alternatively or in addition, the user interface 702 may comprise a dedicated lighting control unit comprising a dedicated actuator or control panel for controlling the light emitting elements 606 within the environment 600. For example, the lighting control device 610 may be in the form of a dedicated control unit (wired or wireless) which can be manually operated by the user 602, e.g. by using one or more buttons, sliders, switches and/or dials of the dedicated control unit.

Alternatively or additionally, the user interface 702 may comprise a microphone for receiving a voice command from the user 602. In another example, the user interface 702 may comprise a camera, infrared detector or the like for detecting gesture commands from the user.

The lighting control device 610 comprises the controller 704 coupled to the user interface 702 in order to receive an indication of the user's commands. The controller 704 is also operatively coupled to a lighting system 706 comprising the one or more light emitting elements 606 discussed in relation to Figure 6 via a wireless transceiver 708. The controller 704 can thereby control the lighting system 706 based on the identified commands in order to select a particular program. For example, the user 602 may select between programs of different time scales, e.g. a first program may emit the periods of illumination according to embodiments of the invention over a time period of twenty four hours, whilst a second program may emit the periods of illumination over a time period of twelve hours. The controller 704 is configured to retrieve the one or more settings from a database 710, e.g. in response to an input to the user interface 702.

In embodiments, the controller 704 is implemented in the form of software stored in memory and a processor arranged for execution the software (the memory on which the software is stored comprising one or more memory units employing one or more storage media, e.g. EEPROM or a magnetic drive, and the processor on which the software is run comprising one or more processing units). Alternatively it is not excluded that some or all of the controller 704 could be implemented in dedicated hardware circuitry, or configurable or reconfigurable hardware circuitry such as a PGA or FPGA. Whatever form it takes, in embodiments the controller 704 may be implemented internally in a single lighting control device 610 along with the user interface 702 and a wireless transceiver 708, i.e. in the same housing. Alternatively the controller 704 could, partially or wholly, be implemented externally such as on a central lighting system bridge 612 or server 710 comprising one or more server units at one or more geographic sites (not shown). The controller 704 may also be partially or wholly implemented on one or more light emitting elements 606.

The controller 704 may be configured to perform some or all of the actions of the lighting control device 610 disclosed herein. For example, the controller 704 is configured to receive the user commands via the user interface 702. The controller 704 is also configured to communicate with one or more light emitting elements 606 within the environment 600 via the wireless transceiver 708 and/or where applicable, the controller 704 is also configured to communicate with the central lighting system bridge 610 or server 710 via the wireless transceiver 708.

The lighting control device 610 comprises the wireless transceiver 708 for communicating via any suitable wireless medium, e.g. a radio transceiver for communicating via a radio channel (though other forms are not excluded, e.g. an ultrasound or infrared transceiver). The wireless transceiver 708 may comprises a Wi-Fi, ZigBee, Bluetooth, Thread etc. interface for communicating with the light emitting elements 606. Each light emitting element 606 may be configured to be able to communicate over a wireless channel in order to perform the respective control operations disclosed herein, preferably a radio channel (though the possibility of other media such as visual light communications, ultrasound or infrared are not excluded). For instance the radio channel may be based on a radio access technology such as ZigBee, Bluetooth, Wi-Fi, Thread, JupiterMesh, Wi-SUN, 6LoWPAN, etc. The radio channel can be used by the lighting control device 610 to control the light emitting elements 606. In the case of receiving commands direct from the lighting control device 610, the light emitting elements 606 each comprise a respective wireless receiver or transceiver (not shown) for connecting directly to the control device 610.

Alternatively, the wireless transceiver 708 may communicate with the light emitting elements 606 via the central lighting system bridge 612 or via a server 710, for example, over a local area network or a wide area network such as the internet. Thus, the light emitting elements 606 may also receive the control commands via the central lighting system bridge 612 or server 710 via a wireless connection.

It is also not excluded that a wired connection could alternately, or additionally, be provided between the central lighting system bridge 612, in wired systems more frequently referred to a central controller instead of a bridge, and the light emitting elements 606 for control purposes, e.g. an Ethernet or DMX connection.

In embodiments the functionality of the central lighting system bridge 612 or server is implemented in the form of software stored in memory and a processor arranged for execution the software (the memory on which the software is stored comprising one or more memory units employing one or more storage media, e.g. EEPROM or a magnetic drive, and the processor on which the software is run comprising one or more processing units). Alternatively it is not excluded that some or all of the functionality of the central lighting system bridge 612 or server could be implemented in dedicated hardware circuitry, or configurable or reconfigurable hardware circuitry such as a PGA or FPGA. Also note again that the central lighting system bridge 612 or server 710 may be implemented locally within the environment 600 or at a remote location, and may comprise one or more physical units at one or more geographic sites.

The central lighting system bridge 612 may comprise a wireless transceiver. The wireless transceiver may comprise a Wi-Fi, ZigBee, Bluetooth, Thread etc. interface for communicating with the light emitting elements 606, lighting control device 610 or server 710 over a local and/or wide area network. For instance a radio channel may be based on a radio access technology such as ZigBee, Bluetooth, Wi-Fi, Thread, JupiterMesh, Wi-SUN, 6LoWPAN, etc. Alternatively or additionally, in embodiments the central lighting system bridge 612 may comprise a wired connection for communicating with the light emitting elements 606, lighting control device 610 or server 710.

Whatever implementation the controller 704 takes in terms of physical infrastructure, the controller 704 on the control device 610, the bridge 612, the light emitting elements 606 and/or the server 710 may be configured to control the light emitting elements 606 in accordance with the following.

The controller 704 is configured to control one or more light emitting elements 606 to emit illumination having a first spectral composition for a first time period. The illumination having the first spectral composition produces a first illumination level on one side of a filter (e.g. inside the unhatched bird eggs 608) that is illuminated by the illumination. Subsequently, the controller 704 controls one or more light emitting elements 606 to emit illumination having a second, different spectral composition for a second time period. The illumination having the second spectral composition produces a second, different illumination level on the same side of the filter (e,g. inside the unhatched bird eggs 608) that is illuminated by the illumination. Herein, illumination level may be an illuminance level, i.e. a measure of illuminance due to the light received directly or indirectly from the light emitting elements. For humans, this means that the spectral power distribution (SPD) is weighted with the V lambda (eye) sensitivity function for photopic vision (in humans). However, for a different species (e.g. chickens) this means the SPD is weighted with the photopic eye sensitivity of that species (for example, the Nuboer curve for chicks) The controller may be configured to account for the weighting of the eye sensitivity of the relevant animals within the environment in order to create the different illuminance levels.

A period of relative darkness or relative lightness followed by a period of relative lightness or relative darkness respectively is beneficial to the unborn birds as it can entrain a circadian rhythm, reduce stress and improve development of the unborn birds.

Here, the spectral composition of the illumination is defined as the range of wavelengths that make up the illumination. The first and second spectral compositions comprise one or more different wavelengths. The first and second spectral compositions may overlap. That is, they may comprise one or more of the same wavelengths. Alternatively, the first and second spectral compositions may be mutually exclusive. That is, the first spectral composition may not comprise any wavelengths included in the second spectral composition. In some examples, the first spectral composition and second spectral composition may be chosen such that the first illuminance level within the unhatched bird eggs 608 is greater than the second illuminance level. Alternatively, the first spectral composition and second spectral composition may be chosen such that the first illuminance level within the unhatched bird eggs 608 is less than the second illuminance level.

The controller 704 is configured to work with a particular filter or type of filter. That is, the controller 704 is configured to set the spectral composition of the emitted illumination based on the particular filter, i.e. to set the spectral composition for the particular filter material (given its transmittance) such that periods of relative lightness and darkness on one side and minimum illumination level on the other side are achieved for that particular filter material. For example, the spectral compositions used for a filter such as an egg shell may be different compared to the spectral compositions used for a filter such as a sunglasses lens. Furthermore, the controller 704 may be configured to change the spectral compositions based on the particular type (species) of animal on each side of the filter, i.e. to set the spectral compositions for the given filter material transmittance and animal type, such that the specified illumination levels are a achieved within certain range of the peak of the spectral sensity curve of the particular species' or animal's visual system. For example, the spectral compositions used for humans may be different compared to the spectral compositions used for chicks.

The first and second time periods may be contiguous. That is, the second time period of illumination begins immediately following the first time period of illumination.

The first and second time periods of illumination may apply to some or all of the same light emitting elements 606. Alternatively, a different set of light emitting elements 606 may be used to emit the first time period of illumination compared to the second time period of illumination. In some examples, a single light emitting element may be used to emit one or both of the time periods of illumination.

The first and second time periods of illumination may have the same duration. That is, the illumination having the first spectral composition is emitted for the same amount of time as the illumination having the second spectral composition. Alternatively, the first and second time periods of illumination may have different durations. For example, the first time period of illumination may have a longer or shorter duration than the second time period of illumination.

The controller 704 is also configured to control one or more of the light emitting elements 606 to emit illumination throughout the first and second time periods to maintain a minimum illuminance level within the environment 600 on a different side of the filter than the side where the time periods of relative lightness and relative darkness are perceived (e.g. outside of the unhatched birds eggs 608 in the environment 600 used by hatched birds 604, other animals, farmers 602, etc.). The illumination emitted throughout the first and second time periods may be the illumination having the first spectral composition followed by the illumination having the second spectral composition (i.e. no additional illumination is emitted). Illumination is emitted continually such that there is always a minimum illuminance level within the environment 600.

Maintaining a minimum level of illumination within the environment 600 is beneficial as it ensures that newborn birds 604 can find food, water and shelter upon hatching. A minimum illumination level is also useful for other observers, e.g. for farmers inspecting the eggs 608 or chicks.

The minimum illuminance level may vary throughout the first and second time periods. For example, the minimum illuminance level maintained during the first time period may be different compared to the minimum illuminance level maintained during the second time period. Alternatively the controller 704 may be configured to maintain a constant illuminance level throughout the first and second time periods. That is, the amount of illumination emitted by the light emitting elements 606 throughout the first and second time periods does not fluctuate.

The controller 704 may control the one or more lighting elements 606 so that the illumination emitted during the first time period has a first egg transmittance factor (ETF). ETF is equal to the amount of illumination that penetrates the shell of an unhatched bird egg divided by the amount of illumination emitted by the light emitting elements 606. In other words, ETF defines the ratio of the Lm (or Lux) within the egg shell compared to the amount of Lm (or Lux) of the light emitting elements 606. The controller 704 may control the one or more lighting elements 606 so that the illumination emitted during the second time period has a second, different egg transmittance factor. As ETF increases more illumination penetrates the shell and therefore the illumination level within the bird egg increases. The second ETF may be greater than the first ETF such that second illuminance level inside the one or more bird eggs 608 is greater than the first illuminance level. Alternatively, the second ETF may be less than the first ETF such that second illuminance level inside the one or more bird eggs 608 is less than the first illuminance level.

The controller 704 may control the one or more light emitting elements 606 so that the illumination emitted during the first time period has a first egg transmittance per visible lumen, i.e. a first amount of illumination penetrates an egg shell per lumen of visible light emitted by the light emitting elements 606. The controller 704 may also control the one or more light emitting elements 606 so that the illumination emitted during the second time period has a second egg transmittance per visible lumen. The second egg transmittance per visible lumen may be greater than the first egg transmittance per lumen such that the second illuminance level inside the one or more bird eggs 608 is greater than the first illuminance level. Alternatively, the second egg transmittance per visible lumen may be less than the first egg transmittance per lumen such that the second illuminance level inside the one or more bird eggs 608 is less than the first illuminance level.

As an optional feature, the controller 704 may control the one or more light emitting elements 606 to emit illumination having a first luminous intensity during the first time period of illumination and to emit illumination having a second, different luminous intensity during the second time period of illumination. The luminous intensity of the emitted illumination is related to the illumination level experienced within the bird eggs 608 such that a greater luminous intensity results in a greater illumination level. Therefore a period of relative lightness can be made relatively lighter or relatively darker by increasing or decreasing the luminous intensity respectively. Similarly, a period of relative darkness can be made relatively lighter or relatively darker by increasing or decreasing the luminous intensity respectively.

In some examples, the first time period of illumination may be divided into two or more sub-periods of illumination, with each sub-period of illumination having a different luminous intensity. For example, the first time period of illumination may be divided into two sub-periods of illumination, with the first sub-period of illumination having a greater luminous intensity compared to the second sub-period of illumination. Therefore the first time period of illumination will have two different illuminance levels. As an example, the first time period of illumination may be a period of relative darkness, with the first sub- period being lighter (or darker) than the second sub-period. Similarly, the second time period of illumination may be divided into two or more sub-periods of illumination, with each sub-period of illumination having a different luminous intensity. By way of example, the second time period of illumination may be a period of relative lightness, with a first sub-period being lighter (or darker) than a second sub-period.

The controller 704 may control the one or more light emitting elements 606 so that the illumination emitted during the first time period is emitted at a first colour temperature. Similarly, the controller 704 may control the one or more light emitting elements 606 so that the illumination emitted during the second time period is emitted at a second, different colour temperature. Since warmer light (lower colour temperature) has been found to be transmitted through bird egg shells more easily (i.e. to a larger extent) than colder light (higher colour temperature), the illuminance level within the bird eggs 608 will decrease as the colour temperature of illumination increases. The second colour temperature may be higher than the first colour temperature such that the second illuminance level inside the one or more bird eggs 608 is lower than the first illuminance level. Alternatively, the second colour temperature may be lower than the first colour temperature such that the second illuminance level inside the one or more bird eggs 608 is higher than the first illuminance level.

In alternative embodiments, the controller 704 may control the one or more light emitting elements 606 so that the illumination emitted during the first time period and the second time period are emitted at the same colour temperature. That is, the colour temperature of the emitted illumination is constant throughout the first and second time periods. Here, the illumination within the unhatched bird eggs 608 is varied by way of the differing spectral compositions of the first and second time periods or by a combination of the differing spectral compositions and differing luminous intensities of light emitted during the first and second time periods of illumination.

The first and second time periods of illumination may form a light-dark cycle of contiguous periods of illumination. That is, the first and second time periods of illumination may repeat periodically, one after the other. The controller 704 may control the light emitting elements 606 to emit illumination having the first spectral composition for the first time period subsequent to emitting the illumination having the second spectral composition for the second time period, and followed by illumination having the second spectral composition, and so on.

The light-dark cycle may comprise at least one further time period of illumination having a third spectral composition that is different compared to the illumination emitted for the first and second time periods of illumination. The third spectral composition may partially overlap with the first and/or second spectral compositions. Alternatively, the third spectral composition may not comprise any wavelengths of light that are present in the first and second spectral compositions. In some examples, the light-dark cycle may comprise additional time periods of illumination, with each time period of illumination having a spectral composition that is different compared to that of the previous time period of illumination. Optionally, each time period of illumination may have a spectral composition that is different compared to that of all of the previous time periods of illumination.

Additionally or alternatively, the light-dark cycle may comprise contiguous periods of illumination that each have a different luminous intensity compared to that of some or all of the other time periods of illumination. That is, each time period of illumination of the light-dark cycle may differ in luminous intensity compared to the previous time period of illumination and/or all of the previous time periods of illumination. For example, the light-dark cycle may comprise at least one further time period of illumination having a third luminous intensity that is different compared to the illumination emitted for the first and second time periods of illumination.

In preferred examples, the light cycle is one day. The light cycle may be exactly or approximately twenty-four hours. In alternative examples, the light cycle may be greater than or less than one day. For example, the light cycle may repeat twice every twenty four hours. In another example, the light cycle may be between twenty and twenty eight hours.

Figure 8 illustrates an example light-dark cycle 800 having four contiguous time periods of illumination. As shown in Figure 8, the spectral compositions (and optionally the illuminous intensities and/or colour temperatures) of the four time periods of illumination are chosen such that the first 801 and third 803 time periods of illumination are periods of relative darkness whilst the second 802 and fourth 804 time periods of illumination are periods of relative lightness.

One, some or all of the time periods of illumination, spectral compositions, luminous intensities and/or colour temperatures may be determined by factors such as, for example, the bird type (e.g. chicken, turkey, duck, etc.), the bird breed (e.g. Cobb, Ross, etc.) and the typical hatch time of the bird type and/or breed. In some examples, the time periods may be programmable by an end-user such as a farmer. For example, the end-user may use the lighting control device 610 to program one or more of the time periods. In alternative examples, the end-user 602 may not be able to change the time periods or light characteristics from the initial settings.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A controller (704) configured to control the illumination emitted by one or more light emitting elements (606) within an environment (600) for hosting one or more unhatched animals in respective unhatched animal eggs (608) and a respective egg shell between each of the one or more unhatched animals and the one or more light emitting elements (606), **characterised in that** the controller (704) is configured to:

   control the one or more light emitting elements (606) to emit illumination having a first spectral composition for a first time period, wherein the first spectral composition is composed to produce, on a first side of the egg shell of the one or more unhatched animal eggs (608), a first level of illumination to create a period of relative lightness on the first side of the egg shell;
   control the one or more light emitting elements (606) to emit illumination having a second, different spectral composition for a second, subsequent time period, wherein the second spectral composition is composed to produce, on a first side of the egg shell of the one or more unhatched animal eggs (608), a second, different level of illumination to create a period of relative darkness on the first side of the egg shell; and
   control the one or more light emitting elements (606) to emit illumination throughout the first and second time periods to maintain, on a second side of the egg shell of the one or more unhatched animal eggs (608), a minimum level of illumination.

2. A controller according to claim 1, wherein the first side of the egg shell of the one or more unhatched animal eggs is the inside the one or more unhatched animal eggs (608), wherein the second side of the egg shell of the one or more unhatched animal eggs (608) is the environment (600) outside the one or more unhatched animal eggs (608).

3. A controller according to claim 1, wherein the first side of the egg shell of the one or more unhatched animal eggs (608) is the environment outside the one or more unhatched animal eggs (608), wherein the second side of the egg shell of the one or more unhatched animal eggs (608) is the inside the one or more unhatched animal eggs (608).

4. A controller (704) according to claim 2 or claim 3, wherein the illumination emitted for the first time period has a first egg transmittance factor and the illumination emitted for the second time period has a second, different egg transmittance factor, wherein the egg transmittance factor is defined as a ratio of: (i) an amount of the illumination that penetrates through the shell of the one or more unhatched animal eggs (608) and (ii) an amount of the illumination incident on the shell of the one or more unhatched eggs and emitted by the one or more light emitting elements (606).

5. A controller (704) according to any of claims 2 to 4, wherein the illumination emitted for the first time period has a first egg transmittance per visible lumen and the illumination emitted for the second time period has a second, different egg transmittance per visible lumen.

6. A controller (704) according to claim 2 or any of claims 4 to 5 when depending on claim 2, wherein the controller (704) is configured to control the one or more light emitting elements (606) to emit illumination throughout the first and second time periods to maintain, outside the one or more unhatched animal eggs (608), a constant level of illumination.

7. A controller (704) according to any preceding claim, wherein the controller (704) is configured to modify the luminous intensity of the illumination emitted by the one or more light emitting elements (606), wherein the illumination emitted for the first time period has a first luminous intensity and the illumination emitted for the second time period has a second, different luminous intensity.

8. A controller (704) according to any preceding claim, wherein the controller (704) is configured to:

control the one or more light emitting elements (606) to emit the illumination in the first time period with a first correlated colour temperature for the first time period; and
control the one or more light emitting elements (606) to emit the illumination in second time period with a second, different correlated colour temperature for the second time period.

9. A controller (704) according to claim 8 when dependent on claim 2 or claim 3, wherein the first correlated colour temperature is greater than the second correlated colour temperature such that the first level of illumination inside or outside the one or more unhatched animal eggs (608) is less than the second level of illumination outside or inside the one or more unhatched animal eggs (608).

10. A controller (704) according to any of claims 1 to 7, wherein the controller (704) is configured to:

control the one or more light emitting elements (606) to emit the illumination in the first time period having a same correlated colour temperature for the first time period; and
control the one or more light emitting elements (606) to emit the illumination in the second time period having the same correlated colour temperature for the second time period.

11. A controller (704) according to any preceding claim, wherein the controller (704) is configured to control the one or more light emitting elements (606) to emit a light-dark cycle (800) of illumination, wherein the light-dark cycle (800) of illumination comprises a plurality of contiguous time periods of illumination comprising the first and second time periods of illumination, wherein the illumination emitted for each respective time period has a different spectral composition and/or a different luminous intensity compared to the illumination emitted for the other respective time periods in the light-dark cycle (800).

12. A controller (704) according to claim 11, wherein the controller (704) is configured to control the one or more light emitting elements (606) to emit the light-dark cycle (800) of illumination over a time period of one day.

13. A controller (704) according to any of claims 11 to 12, wherein the light-dark cycle (800) of illumination comprises at least one further time period of illumination having a third, different spectral composition and/or a third, different luminous intensity compared to the first and second time periods.

14. A system (700) comprising:

a controller (704) according to any of claims 1 to 13; and
one or more light emitting elements (606) configured to emit illumination into the environment (600).

15. A computer program product comprising code embodied on computer-readable storage and configured so as when run on one or more processing units of a control system to control one or more light emitting elements (606) to perform operations in accordance with any of claims 1 to 13.

**Patentansprüche**

1. Steuerung (704), die konfiguriert ist, um die Beleuchtung, die durch ein oder mehrere lichtemittierenden Elemente (606) emittiert wird, innerhalb einer Umgebung (600) zum Beherbergen eines oder mehrerer ungeschlüpfter Tiere in jeweiligen ungeschlüpften Tiereiern (608) und einer jeweiligen Eierschale zwischen jedem des einen oder der mehreren ungeschlüpften Tiere und das eine oder die mehreren lichtemittierenden Elemente (606) zu steuern, **dadurch gekennzeichnet, dass** die Steuerung (704) konfiguriert ist zum:

Steuern des einen oder der mehreren lichtemittierenden Elemente (606), um Beleuchtung, die eine erste spektrale Zusammensetzung aufweist, für einen ersten Zeitraum zu emittieren, wobei die erste spektrale Zusammensetzung zusammengesetzt ist, um auf einer ersten Seite der Eierschale des einen oder der mehreren ungeschlüpften Tiereier (608) ein erstes Beleuchtungsniveau zu produzieren, um einen Zeitraum relativer Helligkeit auf der ersten Seite der Eierschale zu erzeugen;

Steuern des einen oder der mehreren lichtemittierenden Elemente (606), um Beleuchtung, die eine zweite, unterschiedliche spektrale Zusammensetzung aufweist, für einen zweiten, nachfolgenden Zeitraum zu emittieren, wobei die zweite spektrale Zusammensetzung zusammengesetzt ist, um auf einer ersten Seite der Eierschale des einen oder der mehreren ungeschlüpften Tiereier (608) ein zweites, unterschiedliches Beleuchtungsniveau zu produzieren, um einen Zeitraum relativer Dunkelheit auf der ersten Seite der Eierschale zu erzeugen; und

Steuern des einen oder der mehreren lichtemittierenden Elemente (606), um während des gesamten ersten und zweiten Zeitraums Licht zu emittieren, um auf einer zweiten Seite der Eierschale des einen oder der mehreren ungeschlüpften Tiereier (608) ein Mindestniveau von Beleuchtung aufrechtzuerhalten.

2. Steuerung nach Anspruch 1, wobei die erste Seite der Eierschale des einen oder der mehreren ungeschlüpften Tiereier das Innere des einen oder der mehreren ungeschlüpften Tiereier (608) ist, wobei die zweite Seite der Eierschale des einen oder der mehreren ungeschlüpften Tiereier (608) die Umgebung (600) außerhalb des einen oder der mehreren ungeschlüpften Tiereier (608) ist.

3. Steuerung nach Anspruch 1, wobei die erste Seite der Eierschale des einen oder der mehreren ungeschlüpften Tiereier (608) die Umgebung außerhalb des einen oder der mehreren ungeschlüpften Tiereier (608) ist, wobei die zweite Seite der Eierschale des einen oder der mehreren ungeschlüpften Tiereier (608) das Innere des einen oder der mehreren ungeschlüpften Tiereier (608) ist.

4. Steuerung (704) nach Anspruch 2 oder 3, wobei die Beleuchtung, die für den ersten Zeitraum emittiert wird, einen ersten Ei-Transmissionsfaktor aufweist und die Beleuchtung, die für den zweiten Zeitraum emittiert wird, einen zweiten, unterschiedlichen Ei-Transmissionsfaktor aufweist, wobei der Ei-Transmissionsfaktor als ein Verhältnis definiert ist von: (i) einer Menge der Beleuchtung, die durch die Schale des einen oder der mehreren ungeschlüpften Tiereier (608) dringt, und (ii) einer Menge der Beleuchtung, die auf die Schale des einen oder der mehreren ungeschlüpften Eier auftrifft und durch das eine oder die mehreren lichtemittierenden Elemente (606) emittiert wird.

5. Steuerung (704) nach einem der Ansprüche 2 bis 4, wobei die Beleuchtung, die für den zweiten Zeitraum emittiert wird, eine erste Ei-Transmission pro sichtbarem Lumen aufweist und die Beleuchtung, die für den zweiten Zeitraum emittiert wird, eine zweite, unterschiedliche Ei-Transmission pro sichtbarem Lumen aufweist.

6. Steuerung (704) nach Anspruch 2 oder einem der Ansprüche 4 bis 5, wenn von Anspruch 2 abhängig, wobei die Steuerung (704) konfiguriert ist, um das eine oder die mehreren lichtemittierenden Elemente (606) zu steuern, um während des gesamten ersten und zweiten Zeitraums Beleuchtung zu emittieren, um außerhalb des einen oder der mehreren ungeschlüpften Tiereier (608) ein konstantes Beleuchtungsniveau aufrechtzuerhalten.

7. Steuerung (704) nach einem der vorstehenden Ansprüche, wobei die Steuerung (704) konfiguriert ist, um die Leuchtintensität der Beleuchtung zu verändern, die durch das eine oder die mehreren lichtemittierenden Elementen (606) emittiert wird, wobei die Beleuchtung, die für den ersten Zeitraum emittiert wird, eine erste Leuchtintensität aufweist und die Beleuchtung, die für den zweiten Zeitraum emittierte wird, eine zweite, unterschiedliche Leuchtintensität aufweist.

8. Steuerung (704) nach einem der vorstehenden Ansprüche, wobei die Steuerung (704) konfiguriert ist zum:

Steuern des einen oder der mehreren lichtemittierenden Elemente (606), um die Beleuchtung in dem ersten Zeitraum mit einer ersten korrelierten Farbtemperatur für den ersten Zeitraum zu emittieren; und
Steuern des einen oder der mehreren lichtemittierenden Elemente (606), um die Beleuchtung in einem zweiten Zeitraum mit einer zweiten, unterschiedlichen korrelierten Farbtemperatur für den zweiten Zeitraum zu emittieren.

9. Steuerung (704) nach Anspruch 8, wenn von Anspruch 2 oder 3 abhängig, wobei die erste korrelierte Farbtemperatur derart höher als die zweite korrelierte Farbtemperatur ist, dass das erste Beleuchtungsniveau innerhalb oder außerhalb des einen oder der mehreren ungeschlüpften Tiereier (608) geringer als das zweite Beleuchtungsniveau außerhalb oder innerhalb des einen oder der mehreren ungeschlüpften Tiereier (608) ist.

10. Steuerung (704) nach einem der Ansprüche 1 bis 7, wobei die Steuerung (704) konfiguriert ist zum:

Steuern des einen oder der mehreren lichtemittierenden Elemente (606), um die Beleuchtung in dem ersten Zeitraum, der eine gleiche korrelierte Farbtemperatur aufweist, für den ersten Zeitraum zu emittieren; und Steuern des einen oder der mehreren lichtemittierenden Elemente (606), um die Beleuchtung in dem zweiten Zeitraum, der die gleiche korrelierte Farbtemperatur aufweist, für den zweiten Zeitraum zu emittieren.

**11.** Steuerung (704) nach einem der vorstehenden Ansprüche, wobei die Steuerung (704) konfiguriert ist, um das eine oder die mehreren lichtemittierenden Elemente (606) zu steuern, um einen Hell-Dunkel-Zyklus (800) von Beleuchtung zu emittieren, wobei der Hell-Dunkel-Zyklus (800) von Beleuchtung eine Vielzahl von aneinandergrenzenden Beleuchtungszeiträumen umfasst, umfassend den ersten und den zweiten Beleuchtungszeitraum, wobei die Beleuchtung, die für jeden jeweiligen Zeitraum emittiert wird, eine unterschiedliche spektrale Zusammensetzung und/oder eine unterschiedliche Leuchtintensität im Vergleich zu der Beleuchtung aufweist, die für die anderen jeweiligen Zeiträume in dem Hell-Dunkel-Zyklus (800) emittiert wird.

**12.** Steuerung (704) nach Anspruch 11, wobei die Steuerung (704) konfiguriert ist, um das eine oder die mehreren lichtemittierenden Elemente (606) zu steuern, um den Hell-Dunkel-Zyklus (800) von Beleuchtung über einen Zeitraum von einem Tag zu emittieren.

**13.** Steuerung (704) nach einem der Ansprüche 11 bis 12, wobei der Hell-Dunkel-Zyklus (800) von Beleuchtung mindestens einen weiteren Beleuchtungszeitraum umfasst, der eine dritte, unterschiedliche spektrale Zusammensetzung und/oder eine dritte, unterschiedliche Leuchtintensität im Vergleich zu dem ersten und dem zweiten Zeitraum aufweist.

**14.** System (700), umfassend:

eine Steuerung (704) nach einem der Ansprüche 1 bis 13, und
ein oder mehrere lichtemittierende Elemente (606), die konfiguriert sind, um Beleuchtung in die Umgebung (600) zu emittieren.

**15.** Computerprogrammprodukt, umfassend Code, der auf computerlesbarem Speicher verkörpert und konfiguriert ist, um, wenn er auf einer oder mehreren Verarbeitungseinheiten eines Steuersystems ausgeführt wird, das eine oder die mehreren lichtemittierenden Elemente (606) zu steuern, um den Betrieb nach einem der Ansprüche 1 bis 13 durchzuführen.

**Revendications**

**1.** Organe de commande (704) configuré pour commander l'illumination émise par un ou plusieurs éléments émetteurs de lumière (606) au sein d'un environnement (600) permettant d'héberger un ou plusieurs animaux non éclos dans des oeufs d'animaux non éclos (608) respectifs et une coquille d'oeuf respective entre chacun parmi le ou les animaux non éclos et le ou les éléments émetteurs de lumière (606), **caractérisé en ce que** l'organe de commande (704) est configuré pour :

commander le ou les éléments émetteurs de lumière (606) pour émettre une illumination ayant une première composition spectrale pendant un premier laps de temps, dans lequel la première composition spectrale est composée pour produire, sur un premier côté de la coquille d'oeuf du ou des oeufs d'animaux non éclos (608), un premier niveau d'illumination pour créer un laps de temps de clarté relative sur le premier côté de la coquille d'oeuf ;
commander le ou les éléments émetteurs de lumière (606) pour émettre une illumination ayant une deuxième composition spectrale différente pendant un second laps de temps ultérieur, dans lequel la deuxième composition spectrale est composée pour produire, sur un premier côté de la coquille d'oeuf du ou des oeufs d'animaux non éclos (608), un second niveau d'illumination différent pour créer un laps de temps d'obscurité relative sur le premier côté de la coquille d'oeuf ; et
commander le ou les éléments émetteurs de lumière (606) pour émettre une illumination sur l'ensemble des premier et second laps de temps pour maintenir, sur un second côté de la coquille d'oeuf du ou des oeufs d'animaux non éclos (608), un niveau minimum d'illumination.

**2.** Organe de commande selon la revendication 1, dans lequel le premier côté de la coquille d'oeuf du ou des oeufs d'animaux non éclos est l'intérieur du ou des oeufs d'animaux non éclos (608), dans lequel le second côté de la coquille d'oeuf du ou des oeufs d'animaux non éclos (608) est l'environnement (600) à l'extérieur du ou des oeufs d'animaux non éclos (608).

3. Organe de commande selon la revendication 1, dans lequel le premier côté de la coquille d'oeuf du ou des oeufs d'animaux non éclos (608) est l'environnement à l'extérieur du ou des oeufs d'animaux non éclos (608), dans lequel le second côté de la coquille d'oeuf du ou des oeufs d'animaux non éclos (608) est l'intérieur du ou des oeufs d'animaux non éclos (608).

4. Organe de commande (704) selon la revendication 2 ou la revendication 3, dans lequel l'illumination émise pendant le premier laps de temps a un premier facteur de transmittance d'oeuf et l'illumination émise pendant le second laps de temps a un second facteur de transmittance d'oeuf différent, dans lequel le facteur de transmittance d'oeuf est défini en tant que rapport de : (i) une quantité de l'illumination qui pénètre à travers la coquille du ou des oeufs d'animaux non éclos (608) et (ii) une quantité de l'illumination incidente sur la coquille du ou des oeufs non éclos et émise par la ou les éléments émetteurs de lumière (606).

5. Organe de commande (704) selon l'une quelconque des revendications 2 à 4, dans lequel l'illumination émise pendant le premier laps de temps a une première transmittance d'oeuf par lumen visible et l'illumination émise pendant le second laps de temps a une seconde transmittance d'oeuf différente par lumen visible.

6. Organe de commande (704) selon la revendication 2 ou l'une quelconque des revendications 4 à 5 prise en dépendance de la revendication 2, dans lequel l'organe de commande (704) est configuré pour commander le ou les éléments émetteurs de lumière (606) pour émettre une illumination sur l'ensemble des premier et second laps de temps pour maintenir, à l'extérieur du ou des oeufs d'animaux non éclos (608), un niveau constant d'illumination.

7. Organe de commande (704) selon l'une quelconque revendication précédente, dans lequel l'organe de commande (704) est configuré pour modifier l'intensité lumineuse de l'illumination émise par le ou les éléments émetteurs de lumière (606), dans lequel l'illumination émise pendant le premier laps de temps a une première intensité lumineuse et l'illumination émise pendant le second laps de temps a une deuxième intensité lumineuse différente.

8. Organe de commande (704) selon l'une quelconque revendication précédente, dans lequel l'organe de commande (704) est configuré pour :

   commander le ou les éléments émetteurs de lumière (606) pour émettre l'illumination dans le premier laps de temps avec une première température de couleur corrélée pendant le premier laps de temps ; et
   commander le ou les éléments émetteurs de lumière (606) pour émettre l'illumination dans un second laps de temps avec une seconde température de couleur corrélée différente pendant le second laps de temps.

9. Organe de commande (704) selon la revendication 8 prise en dépendance de la revendication 2 ou de la revendication 3, dans lequel la première température de couleur corrélée est supérieure à la seconde température de couleur corrélée de telle sorte que le premier niveau d'illumination à l'intérieur ou à l'extérieur du ou des oeufs d'animaux non éclos (608) est inférieur au second niveau d'illumination à l'extérieur ou à l'intérieur du ou des oeufs d'animaux non éclos (608).

10. Organe de commande (704) selon l'une quelconque des revendications 1 à 7, dans lequel l'organe de commande (704) est configuré pour :

   commander le ou les éléments émetteurs de lumière (606) pour émettre l'illumination dans le premier laps de temps ayant une même température de couleur corrélée pendant le premier laps de temps ; et
   commander le ou les éléments émetteurs de lumière (606) pour émettre l'illumination dans le second laps de temps ayant la même température de couleur corrélée pendant le second laps de temps.

11. Organe de commande (704) selon l'une quelconque revendication précédente, dans lequel l'organe de commande (704) est configuré pour commander le ou les éléments émetteurs de lumière (606) pour émettre un cycle clair-sombre (800) d'illumination, dans lequel le cycle clair-sombre (800) d'illumination comprend une pluralité de laps de temps contigus d'illumination comprenant les premier et second laps de temps d'illumination, dans lequel l'illumination émise pendant chaque laps de temps respectif a une composition spectrale différente et/ou une intensité lumineuse différente par comparaison avec l'illumination émise pendant les autres laps de temps respectifs dans le cycle clair-sombre (800).

**12.** Organe de commande (704) selon la revendication 11, dans lequel l'organe de commande (704) est configuré pour commander le ou les éléments émetteurs de lumière (606) pour émettre le cycle clair-sombre (800) d'illumination sur un laps de temps d'un jour.

**13.** Organe de commande (704) selon l'une quelconque des revendications 11 à 12, dans lequel le cycle clair-sombre (800) d'illumination comprend au moins un laps de temps supplémentaire d'illumination ayant une troisième composition spectrale différente et/ou une troisième intensité lumineuse différente par comparaison avec les premier et second laps de temps.

**14.** Système (700) comprenant :

un organe de commande (704) selon l'une quelconque des revendications 1 à 13 ; et
un ou plusieurs éléments émetteurs de lumière (606) configurés pour émettre une illumination dans l'environnement (600).

**15.** Produit programme d'ordinateur comprenant un code incorporé sur un stockage lisible par ordinateur et configuré de façon à lorsqu'il est exécuté sur une ou plusieurs unités de traitement d'un système de commande pour commander un ou plusieurs éléments émetteurs de lumière (606) mettre en oeuvre des opérations selon l'une quelconque des revendications 1 à 13.

FIG. 1

FIG. 2

Scatterplot of ETF-Nu vs CIE v

Results include rows where 'DC =' < = 0.007 And 'DC =' > -0.007

*FIG. 3*

EP 3 869 948 B1

Scatterplot of ETF-Nu vs CIE v

Results include rows where 'DC =' < = 0.007 And 'DC =' > -0.007

*FIG. 4*

EP 3 869 948 B1

EP 3 869 948 B1

*FIG. 5*

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018160537 A1

**Non-patent literature cited in the description**

- **NUBOER, J. F. W. et al.** Artificial lighting in poultry houses: Are photometric units appropriate for describing illumination intensities?. *British Poultry Science,* 1992, vol. 33 (1), 135-140